# EUROPEAN PATENT APPLICATION

(11) **EP 1 264 879 A1**
(43) Date of publication of application: **11.12.2002**
(21) Application number: 01202221.6
(22) Date of filing: 08.06.2001
(51) Int. Cl.: C12N 5/06

(54) **Isolation of a cluster of cells from an isolated organ using magnetic particle**

(71) Applicant: Leadd B.V., 2333 AL Leiden (NL)
(72) Inventor: The designation of the inventor has not yet been filed
(74) Representative: Prins, Adrianus Willem

(57) **Abstract**

The invention relates to the field of the isolation of a cluster of cells and more in particular to the isolation of islets of Langerhans. The invention provides a method for the isolation of a cluster of cells from an isolated organ wherein said cluster of cells encompasses a network of vascular tissue. Furthermore the invention provides a method for the isolation and *in vitro* prolongation of a cluster of cells from an isolated organ wherein said cluster of cells encompasses a network of vascular tissue. The invention also provides a method for prolonging the *in vitro* lifespan of a cluster of cells comprising culturing said cluster of cells in a culturing medium comprising an extracellular matrix component which surrounds said cluster of cells *in vivo.*

## Description

The invention relates to the field of the isolation of a cluster of cells and more in particular to the isolation of islets of Langerhans.

World wide approximately 65 million people are suffering from diabetes mellitus, of which 30 % with type 1 diabetes mellitus. Insulin-dependent diabetes mellitus (IDDM) is an increasingly common disease but its treatment is still based on intermittent insulin injections, a method developed in the 1920s. This treatment cannot fully and constantly control metabolic dysfunction in IDDM. The resulting fluctuations of the blood glucose levels are responsible for severe long-term complications such as retinopathy, nephropathy and vascular diseases.

Replacement of a patient's islets of Langerhans either by pancreas transplantation, or by isolated islet transplantation is the only treatment of type-1 diabetes mellitus to achieve an insulin-independent, constant normoglymic state avoiding hypoglycemic episodes (1, 2). Pancreas allotransplantation has a high morbidity and is surgically complex. Unfortunately, clinical islet transplantation is still not as efficient as desired and is faced with the problem of a 50 % failure rate after one or three months (4). This failure rate is influenced by several factors, including immunological rejection. Contaminating tissues such as lymph nodes, vascular tissue, and ductal fragments present in the crude digested islet allografts are a major stimulus for induction of an immune response resulting in acute rejection of islet allograft by allotransplantation (5). This problem is exacerbated by the fact that due to the low yield and low viability of the isolated islets, material from multiple donors has to be used, resulting in a less optimal HLA matching and subsequent immune activation.

A simple, rapid method of islet purification is paramount for large-scale human islet isolation, which is required for successful treatment of diabetes. Over the years a number of purification techniques have been tried to obtain enough islets to successfully treat a patient, varying from negative selection by immunomagnetic beads to separation with fluorescence-activated cell sorters to remove the exocrine tissue. But still the purity achieved with these methods is not high enough and there is no better yield of the islets of Langerhans than with other methods (6-8). Presently only two methods of obtaining purified islets are in common use. The first, hand-picking of islets, is time-consuming which is a problem both for cost of preparation as well as for viability of the isolated islets. The results of the second method, density-gradient separation, are inconsistent since differences between acinar cells and islets are altered by conditions affecting the pancreas procurement or digestion process and the use of density-gradients cause injury to purified islets by osmotic shock and repetitive sheer stress forces during the purification. Most investigators currently utilize some form of density gradient separation during the purification step.

The present invention recognises these problems and discloses a method for the isolation of a cluster of cells which encompasses a network of vascular tissue. As one of the main representatives of such a cluster of cells, we describe the islets of Langerhans in greater detail as applications of the present invention. Furthermore the invention provides a method to prolong the lifespan of isolated clusters, preferably by a method according to the invention, of cells by *in vitro* culture on appropriate extracellular matrix components.

In one embodiment the invention provides a method for the isolation of a cluster of cells from an isolated organ wherein said cluster of cells encompasses a network of vascular tissue, comprising:
- capturing a particle in said network of vascular tissue encompassed by said cluster of cells, wherein said particle is of a size capable of being carried through a blood vessel connected to said isolated organ and wherein said size renders said particle capable of being retained by the network of vascular tissue encompassed by said cluster of cells and wherein said particle is further provided with a means to isolate said cluster of cells from said organ
- releasing said cluster of cells from the extracellular matrix surrounding said cluster of cells
- using said means of said particle to isolate said cluster of cells.

Preferentially a method according to the invention is used to isolate a cluster of cells from an isolated organ but a partially isolated organ can also be used. A method for the isolation of a cluster of cells can also be performed on an organ that is still connected to other organs or to other tissues.

A particle which is used in a method for the isolation of a cluster of cells from an isolated organ wherein said cluster of cells encompasses a network of vascular tissue, has to have a certain size which is dependent on the size of the blood vessels connected to the organ of interest and also dependent on the size of the blood vessel comprising the network of vascular tissue encompassed by said cluster of cells. The size of a particle must be small enough to allow a particle to enter the organ of interest via a blood vessel connected to the organ and big enough to allow a particle to be retained in the network of vascular tissue encompassed by said cluster of cells. Furthermore, a particle that is used in a method according to the invention is provided with a means to isolate said cluster of cells. As an example such a particle used in a method according to the invention is provided with a means rendering said particle magnetic or magnetizable. A cluster of cells comprising such a magnetic or magnetizable particle is then isolated with help of magnetic retraction. In a preferred embodiment such magnetic means is provided by a particle that comprises iron (oxide) or cobalt. In yet another example a particle used in a method according to the invention is provided with a means which provides an increase in the mean weight of said cluster of cells. The mean weight of a cluster of cells is for example increased by a particle that comprises gold or another, preferably inert, material with high specific gravity. When the network of vascular tissue is provided with particles which increase the mean weight of a cluster of cells, the cluster of cells sediment much faster in, for example, an isotonic fluid (such as normal growth medium) and thereby preventing the use of Percoll gradients with lengthy incubations to separate a cluster of cells from the remaining (exocrine) tissue of the organ of interest. As yet another example a particle used in a method according to the invention comprises, a for example polymeric, chain which is long enough, after the capturing of a particle in the network of vascular tissue, to emerge from the network of vascular tissue to the outside of a cluster of cells. Attached to the end of the chain is for example a compound like biotine. Immunological retraction with streptavidine is used to isolate such a cluster of cells from the remaining tissue. The prior art provides a lot of these matching immunological compounds and the person skilled in the art knows how to select the appropriate ones. As another example a particle used in a method according to the invention is provided with an amphoteric property. After such a particle is captured in the network of vascular tissue, a change in the pH of the fluid surrounding that particle results in a change of charge, for example from no charge to a negative charge. Applying an opposite charge (in this example a positive charge) allows easy isolation of a cluster of cells comprising such a negatively charged particle.

Even more preferably a particle used in a method according to the invention further comprises biodegradable material. By providing a particle with biodegradable material a particle is more easily removed and reactions of clusters of cells to the presence of for example iron oxide, cobalt or gold are reduced or more preferably completely prevented. Examples of biodegradable material are polylactide, gelatine or starch. During the culturing of the islets, as described to, for example, remove the last remainders of exocrine tissues, the biodegradable matrix is degraded. For example amylase is used to remove the biodegradable component starch. This results in the release of for example iron oxide, cobalt or gold, which is consequently removed by changing the medium several times or by simple diffusion. The amount of for example iron oxide, cobalt or gold in the host is therefore declined. It is clear to a person skilled in the art that the overall size of particles does not have to be completely homogeneous as long as enough particles are able to enter the organ of interest and enough particles are able to be retained by the network of vascular tissue encompassed by a cluster of cells.

After capturing a particle in the network of vascular tissue encompassed by a cluster of cells, a cluster of cells is released from the extracellular matrix (ECM) surrounding said cluster of cells, preferably by treating the organ with an enzyme capable of (partially) degrading the extracellular matrix of the particular organ. In a preferred embodiment said enzyme capable of degrading the ECM surrounding cluster of cells is a protease and even more the protease is a collagenase. Preferably a cluster of cells is completely released from the surrounding extracellular matrix but extracellur matrix components still being linked to a cluster of cells can be removed by subsequent tissue culturing steps. It is clear to a person skilled in the art that a cluster of cells is also released from the extracellular matrix by other enzymes than collagenase. The composition of the ECM differs per organ and the person skilled in the art knows how to select the right enzyme or enzymes for each organ.

After capturing a particle in the network of vascular tissue encompassed by a cluster of cells and after releasing a cluster of cells from their surrounding extracellular matrix a cluster of cells is isolated by for example magnetic or immunological retraction or by sedimentation. As disclosed herein within the experimental part the clusters of cells isolated according to a method of the invention are either more pure, or the isolation is less labour intensive or less time consuming compared to methods described in the prior art.

In a preferred embodiment a method according to the invention is used to isolate islets of Langerhans from a preferentially isolated pancreas. Approximately 5 percent of the total pancreatic mass is comprised of endocrine cells. The endocrine cells are clustered in groups within the pancreas which look like little islets of cells when examined under a microscope. Within pancreatic islets (islets of Langerhans) are cells which make specific pancreatic endocrine hormones, of which the best-known is insulin. The produced hormones need to be transported and therefore the pancreatic islets are vascularized by a network of capillaries with diameters in the range of 6-10 µm (in rat) (9, 10).

In another embodiment the invention provides a cluster of cells obtainable by a method according to the invention and even more preferable the invention provides isolated islets of Langerhans.

With such isolated islets of Langerhans the invention provides use of said islets for transplantation purposes and a method for treating an individual with diabetes mellitus comprising providing said individual with isolated islets of Langerhans. Preferably said islets are allogeneic (from the same species), although islets originating from another species are also used (xenogeneic). An example of xenogeneic transplantation is the use of islets of Langerhans isolated from a pig's pancreas which are transplanted into a human in need for such islets due to diabetes mellitus. It is also important to notice that not all particles captured by the network of vascular tissue have to be removed after the isolation of said islets because after transplantation new vascular tissue is formed within the transplanted islets of Langerhans.

In yet another embodiment the invention provides a method for the isolation and *in vitro* prolongation of a cluster of cells from an isolated organ wherein said cluster of cells encompasses a network of vascular tissue comprising:
- a method for the isolation of said cluster of cells according to the invention
- culturing said isolated cluster of cells in a culturing medium comprising an extracellular matrix component which surrounds said cluster of cells *in vivo*. Preferably said cluster of cells comprises islets of Langerhans and even more preferably said extracellular matrix component comprises laminin and/or collagen type IV. As disclosed herein within the experimental part, between the beta cells of the islets of Langerhans and the surrounding tissue, mostly laminin and collagen type IV is present, whereas in between the islets collagen type I was found to be a major component of the ECM. Indeed, when cultured on collagen type IV or laminin, the islets were still viable after 24 hrs, in contrast with islets cultured on polyHEMA (which prevents the deposition of any extracellular material at all) or collagen type I. When cultured in the absence of the appropriate ECM materials, 50% of these islets had even disappeared after 24 hrs. By providing a culturing medium for example for islets of Langerhans with laminin and/or collagen type IV adhesion and/or survival of the islets is allowed.

In another embodiment the invention provides a method for prolonging the *in vitro* lifespan of a cluster of cells comprising culturing said cluster of cells in a culturing medium comprising an extracellular matrix component which surrounds said cluster of cells *in vivo.* More preferably said cluster of cells comprises islets of Langerhans and even more preferably said extracellular matrix component comprises laminin and/or collagen type IV. The present invention discloses that the extracellular matrix that is present in a liver comprises components, which are comparable to the extracellular matrix components surrounding the islets of Langerhans. Furthermore, culturing isolated hepatocytes on media comprising laminin and/or collagen type IV resulted in a dramatically increased percentage of survival compared to culturing conditions described in the prior art. Therefore it is expected that cells or clusters of cells can be cultured *in vitro* on or in media comprising the extracellular components which surround said cells or clusters of cells *in vivo*.

Currently, transplantation of islets of Langerhans cells is accompanied by a period of a couple of days (waiting period or "preparing time-period") between the isolation of the islets of Langerhans and the actual transplantation. During this waiting period the survival of islets decreases dramatically. The method for prolonging the *in vitro* lifespan of a cluster of cells comprising culturing said cluster of cells in a culturing medium comprising an extracellular matrix component which surrounds said cluster of cells *in vivo* provided by the present invention increases the survival of the islets of Langerhans in this waiting period.

The extracellular matrix component(s) in the medium used for culturing isolated cells or isolated clusters of cells are provided either as a solid layer or as structures which should be large enough to provide the receptors on the outside of the cluster of cells with the capability to attach to the extracellular matrix.

The invention will be explained in more detail in the following detailed description, which is not limiting the invention.

### EXPERIMENTAL PART

### 1. Isolation of pancreatic islet cells with iron oxide crystals and magnetic separation.

### 1.1. Preparation and characterization of iron oxide particles

1.25 % Fe₃O₄ (Serva) w/v in phosphate buffered saline (PBS) was sonicated for 30 seconds in a Branson sonicator.

### 1.2. Perfusion and digestion of the pancreas

Inbred male rats (weighing 200-250 g) of the Wistar strain were anaesthetised by a subcutaneous injection of a mixture of ketamin, thalomonal and hypnorm in phosphate buffered saline. The abdominal wall was opened by midline incision.

The aorta was perfused with sonicated iron oxide after ligation of the thoracic aorta. An incision in the distal vena cava was carried out to provide outflow of fluid. Subsequently 10 ml of 0.7 mg/ml collagenase P (Boehringer Mannheim, The Netherlands) in 25 mM Krebs-Ringer-HEPES (KRH) containing 10% BSA (Sigma-Aldrich, The Netherlands) was slowly introduced into the cannulated common bile duct after ligation of the distal end just proximal to the duodenum. The pancreas was excised and the digestion was continued in a water bath at 37°C for 15 min. The digest was passed through a metal screen with a pore diameter of 210 µm. The obtained effluent was passed through a 50 µm pore diameter metal screen and the residue was collected.

Alternatively, the described method is also applicable on isolated organs whereby the particles comprising the right size and properties are introduced via a blood vessel connected to said organ.

### 1.3. Isolation of the pancreatic islets

Using a Dynal Magnetical Partical Concentrator (Dynal AS, Oslo, Norway) the islets were purified. The falcon tube was placed immediately next to the magnetic pole for 30 sec. Successful magnetic retraction was noted by visible accumulation of black specks on the inner sidewall of the tube next to the magnet. The islets were subsequently collected in CMRL 1066 culture.

### 2. Characterization of pancreatic islets and hepatocytes

### 2.1 Determination of the yield of pancreatic islets

By means of a Buerker-Tuerker cell counter/hemocytometer the amount of isolated harvested islets, being clusters of 50-60 cells, was determined. The percentage of harvested isolated pancreatic islets was determined by the amount of counted islets divided by 2000 (in general, the pancreas of a rat contains 2000 islets) and multiplied with 100.

### 2.2 Determination of in vitro culture conditions for optimal islet viability and function

To prove the viability of isolated rat pancreatic islets, 100 pancreatic islets were incubated in CMRL 1066 culture medium in a 24-wells dish (Greiner, FRG) coated with collagen IV and laminin. After 24 hours, the amount of intact pancreatic islets was determined by means of a phase-contrast microscope.

Furthermore, aliquots were taken to determine the viability of the islets as well as their apoptotic characteristics were determined.

### 2.3 Detection of cell viability and integrity with trypan-blue exclusion

Trypan-blue exclusion is a cell viability assay based on the ability of live cells to exclude the dye trypan blue. With this method one can analyse cells undergoing late stages of apoptosis and/or necrosis.

To assess the viability of the isolated islets, an equal volume of trypan blue dye solution (0.1% w/v) was added to an aliquot of approximately 100 isolated islets and was allowed to sit briefly at room temperature to allow for dye uptake. The isolated rat pancreatic islets were then loaded into a hemocytometer and scored for dye uptake. An islet was scored positive when at least one cell of the islet was stained with trypan blue.

The same procedure was used for the determination of viable isolated rat hepatocytes.

For propidiumiodide exclusion, hepatocytes or pancreatic islet cells were first incubated with 5 µg of propidiumiodide per ml in the medium for 20 minutes, washed with Phosphate-buffered saline, and then fixed with formaldehyde-methanol-acetone (sequential incubation with 1% formaldehyde for 10 minutes, 100% cold methanol for 5 minutes and 80% cold acetone for 2 minutes). The cells were examined by means of a fluorescence microscope.

### 2.4 Detection of apoptotic cell death

Isolated rat hepatocytes or isolated pancreatic islets were examined for their apoptotic activity by means of a caspase-3 assay. Caspase 3 is one of the crucial apoptotic executioner caspases, and essential in apoptosis of hepatocytes and cells derived from pancreatic islets.

Hepatocytes or pancreatic islet cells were lysed in standard cell-lysis buffer and subsequently four times freeze-thawed. The protein content was determined according Bradford and 10 µg protein was mixed with 25 µM Ac-DEVD-AMC, which is the substrate for caspase 3. The caspase 3 cleavage of the substrate results in a fluorescent product. The enzymatic reaction was followed for 1.5 hours by measuring the amount of fluorescent signal by means of a Wallac apparatus.

Another caspase-3 assay was also used. Hepatocytes and pancreatic islet cells were fixed with 80% acetone for 10 minutes and stained with a rabbit polyclonal antibody to detect active caspase-3 (dilution 1:200, Pharmingen, San Diego, CA, USA) as described by the supplier.

### 2.5 Detection of the insulin-production after glucose challenge by means of RIA.

One-hundred isolated islets were incubated in CMRL-1066 culture medium and challenged with glucose according standard procedures of the Laboratory of Clininal Chemistry, Leiden University Medical Center, Leiden, The Netherlands headed by Dr Froehlich.

Antibodies directed against insulin were used and were obtained from Amersham, UK. The actual insulin examinations were carried out by means of an Analyzer of the Laboratory of Clinical Chemistry.

### 3. Characterization of the presence of iron crystals in the isolated islets.

Pancreatic islets were treated with iron oxide crystals and isolated by means of magnetic separation. By means of a phase-contrast microscope, magnification of 400 x, the iron crystals in the blood vessels of the isolated islets were determined

### 4. Isolation of hepatocytes

Primary rat hepatocytes were isolated from collagenase-perfused livers as standard and cultured in Williams E medium (Gibco Life Technologies, Grand Island, NY, USA) supplemented with insulin (2 mU/ml) and dexamethasone (1 mM).

### RESULTS

### Determination of the size of iron oxide crystals

We have determined that the iron oxide particles used in our experiments have a particle range between 50 and 400 nm as measured by calibrated Electron Microscopy.

### Characterization of the isolated islets

The isolation of pancreatic islets by means of the methods known thus far resulted in at most 10% of the total amount of islets present in the pancreas. The herein described methods comprise several improvements compared to the prior art methods. Furthermore, the viability of the islets is also rather poor. This is of importance, because these islets will finally not be able to produce insulin.
By means of a hemocytometer the percentage of isolated pancreatic islets obtained with the iron-oxide crystal method, was repeatedly found to be around 60% of the total amount of rat pancreatic islets. With the here described new method almost all if not all of the contaminating exocrine tissue was proven to be removed. Also of importance, the isolation period is at least four times faster than using a density-gradient and subsequent handpicking of the islets.

The described isolation method was proven to be very effective, for almost all isolated islets were proven to be intact after an 24h incubation. By means of trypan-blue and/or propidium-iodide exclusion assays and an apoptotic cell death assay, it was shown that the far majority of the isolated islets was viable and not prone to undergo cell death.

Therefore, we conclude that the herein described method harbours several improvements compared to the known ones. Thus far 3 different donors were required to treat 1 patient, which created severe problems. For instance, severe immunogenic reactions were induced against the transplanted islets Using the herein described method the amount of isolated islets is dramatically improved, which means that instead of 3 donors per patient, 0.5-1 donor is now sufficient for transplanting a diabetes patient. A method according to the invention is less time-consuming (at least a factor 4), furthermore the absence of the exocrine tissue results in less apoptosis in pancreatic islets and/or less immunogenic responses against the islets upon transplantation.

### Characterization of the presence of iron oxide crystals in the isolated islets

By means of phase-contrast microscopy of section of isolated pancreatic islets it was clearly shown that the iron oxide crystals are present in the vessels of the pancreatic islets. The vessels were coloured black due to the presence of the iron oxide crystals. These data were confirmed by electron-microscopy analysis on isolated islet sections.

### Determination of in vitro culture conditions for optimal islet viability and function

We determined the composition of the extracellular matrix (ECM) which is present around the islets *in vivo,* in order to be able to supply the optimal support for the islets in culture *in vitro.* Between the beta cells of the islets and the surrounding tissue, mostly laminin and collagen type IV was found, whereas in between the islets collagen type I was found to be a major component of the ECM. Indeed, when cultured on collagen type IV or laminin, the islets were still viable after 24 hrs, in contrast with islets cultured on polyHEMA or collagen type I. When cultured in the absence of the appropriate ECM materials, 50% of these islets had even disappeared after 24 hrs. In conclusion, not only the isolation method is important to obtain an optimal yield and viability, but also the *in vitro* culture conditions of the islets after isolation. Currently, transplantation of islets of Langerhans cells is accompanied by a period of a couple of days ("preparing time-period") between the isolation of the islets of Langerhans and the actual transplantation. By means of the use of the required ECM components the survival of islets during the "preparing time-period" is much less dramatically decreased as has been reported for the known procedures.

### Determination of in vitro culture conditions for optimal viability of hepatocytes

We also have determined the composition of the ECM of the rat liver, in order to be able to supply the optimal support for the isolated hepatocytes under culture conditions. Mostly laminin and collagen type IV was found surrounding the hepatocytes.

Indeed, when cultured on collagen type IV or laminin, the hepatocytes were still viable and not prone to undergo apoptosis as measured with the above described assays. In contrast, hepatocytes cultured on polyHEMA or collagen type I (so-called wrong matrix type) underwent massively apoptosis as measured by propidium-exclusion assays or by means of caspase-3 assays.

In conclusion, the *in vitro* culture conditions of the isolated hepatocytes are enormously improved when the hepatocytes are grown on its "own" ECM components. In general, this implies that isolated cells of any tissue kind will survive much longer under cell-culture conditions in the vicinity of their own specific ECM components.

### REFERENCES

1. Bretzel,RG, Brandhorst,D, Brandhorst,H, Eckhard,M, Ernst,W, Friemann,S, Rau,W, Weimar,B, Rauber,K, Hering,BJ, Brendel,MD: Improved survival of intraportal pancreatic islet cell allografts in patients with type-1 diabetes mellitus by refined peritransplant management. *J Mol.Med.* 77:140-143, 1999
2. Shapiro,AM, Lakey,JR, Ryan,EA, Korbutt,GS, Toth,E, Warnock,GL, Kneteman,NM, Rajotte,RV: Islet transplantation in seven patients with type 1 diabetes mellitus using a glucocorticoid-free immunosuppressive regimen. *N.Engl.J.Med.* 343:230-238, 2000
3. Oberholzer,J, Triponez,F, Mage,R, Andereggen,E, Buhler,L, Cretin,N, Fournier,B, Goumaz,C, Lou,J, Philippe,J, Morel,P: Human islet transplantation: lessons from 13 autologous and 13 allogeneic transplantations [In Process Citation]. *Transplantation* 69:1115-1123, 2000
4. Ricordi,C, Tzakis,AG, Carroll,PB, Zeng, YJ, Rilo,HL, Alejandro,R, Shapiro,A, Fung,JJ, Demetris,AJ, Mintz,DH, .: Human islet isolation and allotransplantation in 22 consecutive cases. *Transplantation* 53:407-414, 1992
5. Gotoh,M, Maki,T, Satomi,S, Porter,J, Monaco,AP: Immunological characteristics of purified pancreatic islet grafts. *Transplantation* 42:387-390, 1986
6. Davies,JE, James,RF, London,NJ, Robertson,GS: Optimization of the magnetic field used for immunomagnetic islet purification. *Transplantation* 59:767-771, 1995
7. Davies,JE, Winoto-Morbach,S, Ulrichs,K, James,RF, Robertson,GS: A comparison of the use of two immunomagnetic microspheres for secondary purification of pancreatic islets. *Transplantation* 62:1301-1306, 1996
8. Fujioka,T, Terasaki,PI, Heintz,R, Merideth,N, Lanza,RP, Zheng,TL, Soon-Shiong,P: Rapid purification of islets using magnetic microspheres coated with anti-acinar cell monoclonal antibodies. *Transplantation* 49:404-407, 1990
9. Merchant,FA, Diller,KR, Aggarwal,SJ, Bovik,AC: Angiogenesis in cultured and cryopreserved pancreatic islet grafts. *Transplantation* 63:1652-1660, 1997
10. Heuser,M, Wolf,B, Vollmar,B, Menger,MD: Exocrine contamination of isolated islets of Langerhans deteriorates the process of revascularization after free transplantation. *Transplantation* 69:756-761, 2000
11. Gauthier,VJ, Mannik,M: A method for isolation of mouse glomeruli for quantitation of immune deposits. *Kidney Int.* 33:897-899, 1988
12. Baelde,HJ, Bergijk,EC, Bruijn,JA: Isolation and characterization of mouse glomerular basement membrane. *J.Clin.Lab Immunol.* 33:17-20, 1990
13. Baelde,JJ, Bergijk,EC, Hoedemaeker,PJ, de Heer,E, Bruijn,JA: Optimal method for RNA extraction from mouse glomeruli. *Nephrol.Dial.Transplant.* 9:304-308, 1994

## Claims

1. A method for the isolation of a cluster of cells from an isolated organ wherein said cluster of cells encompasses a network of vascular tissue, comprising:
- capturing a particle in said network of vascular tissue encompassed by said cluster of cells, wherein said particle is of a size capable of being carried through a blood vessel connected to said isolated organ and wherein said size renders said particle capable of being retained by the network of vascular tissue encompassed by said cluster of cells and wherein said particle is further provided with a means to isolate said cluster of cells from said organ
- releasing said cluster of cells from the extracellular matrix surrounding said cluster of cells
- using said means of said particle to isolate said cluster of cells.

2. A method according to claim 1 wherein said particle is provided with a means rendering said particle magnetic or magnetizable.

3. A method according to claim 1 or 2 wherein said means comprises iron oxide.

4. A method according to claim 1 wherein said particle is provided with a means which increases the mean weight of said cluster of cells

5. A method according to claim 1 or 4 wherein said means comprises gold.

6. A method according to anyone of claims 1 to 5 wherein said particle further comprises biodegradable material.

7. A method according to anyone of claims 1 to 6 wherein said cluster of cells is released from the extracellular matrix by an enzyme treatment.

8. A method according to claim 7 wherein said enzyme is a protease.

9. A method according to claim 8 wherein said protease is collagenase.

10. A method according to anyone of claims 1 to 9 wherein said cluster of cells comprises islets of Langerhans.

11. A cluster of cells obtainable by a method according to anyone of claims 1 to 9.

12. Islets of Langerhans obtainable by a method according to anyone of claims 1 to 10.

13. Use of islets of Langerhans according to claim 12 for transplantation purposes.

14. A method for treating an individual with diabetes mellitus comprising providing said individual with islets of Langerhans according to claim 12.

15. A method according to claim 14 wherein said cells are allogenic or xenogenic.

16. A method for the isolation and *in vitro* prolongation of a cluster of cells from an isolated organ wherein said cluster of cells encompasses a network of vascular tissue comprising
- a method for the isolation of said cluster of cells according to anyone of claims 1 to 9
- culturing said isolated cluster of cells in a culturing medium comprising an extracellular matrix component which surrounds said cluster of cells *in vivo*.

17. A method according to claim 16 wherein said cluster of cells comprises islets of Langerhans.

18. A method according to claim 17 wherein said extracellular matrix component comprises laminin and/or collagen type IV.

19. A method for prolonging the *in vitro* lifespan of a cluster of cells comprising culturing said cluster of cells in a culturing medium comprising an extracellular matrix component which surrounds said cluster of cells *in vivo*.

20. A method according to claim 19 wherein said cluster of cells comprises islets of Langerhans.

21. A method according to 20 wherein said extracellular matrix component comprises laminin and/or collagen type IV.
